# EUROPEAN PATENT APPLICATION

(11) **EP 4 206 315 A1**
(43) Date of publication of application: **05.07.2023**
(21) Application number: 21861751.2
(22) Date of filing: 30.08.2021
(51) Int. Cl.: C12N 1/16

(54) **COMPOSITION HAVING REDUCIBILITY**

(30) Priority: 31.08.2020 JP 2020145728
(71) Applicant: Asahi Group Holdings, Ltd., Tokyo 130-8602 (JP)
(72) Inventor: KITAGAWA, Takanori, Moriya-shi, Ibaraki 302-0106 (JP); KATSURAGAWA, Kazuhiko, Seki-shi, Gifu 501-3511 (JP)
(74) Representative: Vossius & Partner Patentanwälte Rechtsanwälte mbB
(86) International application number: PCT/JP2021/031688
(87) International publication number: WO 2022/045330

(57) **Abstract**

[Problem] To provide a new technology that enables reducibility to be maintained for a prolonged period of time.

[Solution] A composition having reducing ability, comprising a substance having reducing ability and a wet ball mill-treated product of yeast cell wall and/or a hydrothermal reaction-treated product of yeast cell wall.

## Description

### Technical Field

The present invention relates to a composition having reducing ability.

### Background Art

Various uses are known for substances having reducing ability to donate electrons to other substances etc., and one of them is the elimination of oxidative stress (active oxygen), for example.

All organisms such as humans, animals, and plants are constantly exposed to oxidative stress, and it is clear that the accumulation of oxidative stress causes aging and lifestyle-related diseases such as cancers and diabetes. In addition, as oxidative stress increases, immunity declines, so it is very important to eliminate oxidative stress (active oxygen) constantly and persistently in order to counter foreign enemies such as the new coronavirus.

A substance having reducing ability has an active oxygen-removing ability (antioxidative ability) by itself, or can impart the active oxygen-removing ability to other substances, etc., depending on the value of its redox potential, etc.

As a substance having reducing ability, for example, the substance described in Patent Literature 1 is known.

### Citation List

### Patent Literature

Patent Literature 1: Japanese Patent No. 4986315

### Summary of Invention

### Technical Problem

An object of the present invention is to provide a novel technique capable of maintaining reducing ability for a longer period of time.

### Solution to Problem

As a result of intensive studies, the present inventors have discovered that reducing ability can be maintained for a longer period of time when a wet ball mill-treated product of yeast cell wall and/or a hydrothermal reaction-treated product of yeast cell wall is contained together with a substance having reducing ability, and thus have completed the present invention.
[1] A composition having reducing ability, comprising a substance having reducing ability and a wet ball mill-treated product of yeast cell wall and/or a hydrothermal reaction-treated product of yeast cell wall.
[2] The composition according to [1], wherein the redox potential of the composition is -300 mV or less.
[3] The composition according to [1] or [2], containing a gelled material obtained by treating a quartz porphyry as the substance by wet ball milling, the quartz porphyry containing silicic acid (SiO₂), zinc (Zn), copper (Cu), aluminum (Al), iron (Fe), calcium (Ca), magnesium (Mg), potassium (K), strontium (Sr), and phosphorus (P).
[4] The composition according to any one of [1] to [3], containing the hydrothermal reaction-treated product.
[5] A method for sustaining reducing ability, comprising forming a composition containing a wet ball mill-treated product of yeast cell wall and/or a hydrothermal reaction-treated product of yeast cell wall together with a substance having reducing ability.
[6] The method according to [5], wherein the redox potential of the composition is -300 mV or less.
[7] The method according to [5] or [6], wherein the substance is the gelled material obtained by treating a quartz porphyry as the substance by wet ball milling, the quartz porphyry containing silicic acid (SiO₂), zinc (Zn), copper (Cu), aluminum (Al), iron (Fe), calcium (Ca), magnesium (Mg), potassium (K), strontium (Sr), and phosphorus (P).
[8] The method according to any one of [5] to [7], wherein the hydrothermal reaction-treated product is contained together with the substance.
[9] An agent for sustaining reducing ability, comprising a wet ball mill-treated product of yeast cell wall and/or a hydrothermal reaction-treated product of yeast cell wall.
[10] The agent for sustaining reducing ability according to [9],comprising a hydrothermal reaction-treated product of yeast cell wall.

### Advantageous Effects of Invention

According to the present invention, a novel technique capable of maintaining reducing ability for a longer period of time can be provided.

### Description of Embodiments

Hereinafter, one embodiment of the present invention will be described in detail.

The embodiment relates to a composition having reducing ability, wherein the composition contains a substance having reducing ability and a wet ball mill-treated product of yeast cell wall and/or a hydrothermal reaction-treated product of yeast cell wall.

The composition of the embodiment has reducing strength that is varied depending on the type of the substance having reducing ability to be used, etc., and as a result, the composition of the embodiment is expected to be utilized in various fields.

Whether or not the substance has reducing ability can be confirmed by, for example, measuring the redox potential and checking whether or not it has a negative redox potential. The measurement can be performed using a general redox potential measuring meter, for example.

The substance having reducing ability is a substance having a redox potential of less than 0 mV (minus), and thus is expected to have an active oxygen-removing ability (antioxidative ability) and an effect of imparting the active oxygen-removing ability to other substances and the like. Many substances having reducing ability are known, but these substances are known to be easily oxidized by surrounding substances and easily lose the reducing ability, depending on the environment.

In one aspect of the present invention, the composition contains a hydrothermal reaction-treated product of yeast cell wall, but does not use the hydrothermal reaction-treated product itself of yeast cell wall as a reducing composition, and instead sustains the reducing property of the substance having reducing ability to be combined, and thereby can have an effect that cannot be obtained by a composition only the hydrothermal reaction-treated product of yeast cell wall. Therefore, it is desirable that the composition having reducing ability of the embodiment has reducing property stronger than that of the hydrothermal reaction-treated product of yeast cell wall. For example, the composition has preferably a redox potential of -300 mV or less, more preferably a redox potential of -500 mV or less, and even more preferably a redox potential of -700 mV or less.

According to the embodiment, a composition having more sustainable reducing ability can be provided. Note that the expression "sustaining reducing ability" used herein means to make it possible to sustain the reducing ability for a longer period of time. The composition of the embodiment can maintain a negative redox potential for a longer period of time, and such a substance is unknown as far as the inventors know, and its various uses are expected described later. For example, it is considered that reducing ability maintained for a longer period of time can lead to the more persistent removal of active oxygen.

Further, it is considered that the lower the potential of a substance having reducing ability, the more easily the reducing ability is lowered. In particular, a substance having reducing ability of -700 mV or less is not well known, and therefore, a substance in which such a negative potential is persistently maintained is considered to be very valuable. These substances are very useful because they can impart the active oxygen removing ability to other substances and the like.

The substance having reducing ability may be composed of a single component or may be a composition, and is not particularly limited. An example of such a substance having reducing ability is a gelled material (hereinafter, also referred to as a gelled quartz porphyry) obtained by treating a quartz porphyry by wet ball milling, the quartz porphyry containing silicic acid (SiO₂), zinc (Zn), copper (Cu), aluminum (Al), iron (Fe), calcium (Ca), magnesium (Mg), potassium (K), strontium (Sr) and phosphorus (P). From the viewpoint of sustainability of reducing ability, the composition of the embodiment preferably contains the gelled quartz porphyry as the substance having reducing ability.

The gelled quartz porphyry can be obtained by the method described in Patent Literature 1, for example.

The term "quartz porphyry" refers to an acidic porphyry (igneous rock) having phenocrysts of quartz and orthoclase. The gelled quartz porphyry can be prepared using a quartz porphyry containing silicic acid (SiO₂), zinc (Zn), copper (Cu), aluminum (Al), iron (Fe), calcium (Ca), magnesium (Mg), potassium (K), strontium (Sr), and phosphorus (P), among quartz porphyries. The gelled quartz porphyry can be obtained by ball milling a quartz porphyry having such a composition, then dispersing the resultant in water in an amount that gives a gel form to obtain a suspension, and then subjecting the suspension to treatment using a wet ball mill, thereby crushing and gelling the quartz porphyry. The obtained gelled quartz porphyry exhibits a negative redox potential and has reducing ability.

The composition of a quartz porphyry can be confirmed by analysis using a fluorescent X-ray analysis method.

A wet ball mill-treated product of yeast cell wall can be obtained by subjecting yeast cell wall to wet ball milling. The conditions and the like for the wet ball mill treatment are not particularly limited, can be appropriately set by those skilled in the art, and can be, for example, the same conditions as those of the above-mentioned treatment for obtaining the gelled quartz porphyry.

The hydrothermal reaction-treated product of yeast cell wall is disclosed in, for example, International Publication No. WO2013/0942535, and can be prepared according to the method disclosed in International Publication No. WO2013/094235. The international application pertaining to International Publication No. WO2013/094235 is registered as Japanese Patent No. 5555818 in Japan, for example.

Yeast cell wall to be used as a raw material for the hydrothermal reaction treatment is not particularly limited, and may be a dry yeast cell wall or a yeast cell wall suspension, for example.

The term "hydrothermal reaction treatment (superheated steam treatment)" as used herein means a method of generating superheated steam by heating and pressurizing, so as to change the physical properties of an object by the influence of the generated superheated steam.

The temperature at which superheated steam is generated is preferably 120°C or higher and 220°C or lower, and more preferably 150°C or higher and 210°C or lower. Further, the pressure for generating superheated steam is preferably 0.9 MPa or more and 1.9 MPa or less, and more preferably 1.2 MPa or more and 1.8 MPa or less. In particular, a hydrothermal reaction treatment performed at a pressure of 0.9 MPa or more and 1.9 MPa or less and a temperature of 120°C or higher and 220°C or lower is preferable, a hydrothermal reaction treatment performed at a pressure of 0.9 MPa or more and 1.9 MPa or less, and a temperature of 150°C or higher and 210°C or lower is more preferable, and a hydrothermal reaction treatment performed at a pressure of 1.2 MPa or more and 1.8 MPa or less and a temperature of 150°C or higher and 210°C or lower is even more preferable.

The composition of the embodiment preferably contains a hydrothermal reaction-treated product of yeast cell wall because the fluctuation of the redox potential is smaller.

The composition of the embodiment can be prepared by forming the composition containing a wet ball mill-treated product of yeast cell wall and/or a hydrothermal reaction-treated product of yeast cell wall together with a substance having reducing ability such as a gelled quartz porphyry. Specifically, a substance having reducing ability and a wet ball mill-treated product of yeast cell wall and/or a hydrothermal reaction-treated product of yeast cell wall may be mixed, for example. The method of mixing, the timing of mixing the substance having reducing ability with the wet ball mill-treated product of yeast cell wall and/or the hydrothermal reaction-treated product of yeast cell wall in the production process of the composition, and the like are not particularly limited, and can be appropriately set by those skilled in the art.

For example, when the composition of the embodiment contains a gelled quartz porphyry and a wet ball mill-treated product of yeast cell wall, the composition can be obtained by mixing the quartz porphyry having the above composition and yeast cell wall, and then subjecting the thus obtained mixture to a wet ball mill treatment.

When the composition of the embodiment contains a gelled quartz porphyry and a hydrothermal reaction-treated product of yeast cell wall, the gelled quartz porphyry having the above composition is treated by wet ball milling, yeast cell wall is treated by hydrothermal reaction, and then the thus obtained gelled quartz porphyry and the hydrothermal reaction-treated product of yeast cell wall may be mixed, for example.

The mixing ratio of the substance having reducing ability and the wet ball mill-treated product of yeast cell wall and/or the hydrothermal reaction-treated product of yeast cell wall is not particularly limited and can be appropriately set by those skilled in the art. On the other hand, from the viewpoint of maintaining the reducing ability, the mixing ratio of the wet ball mill-treated product of yeast cell wall and/or the hydrothermal reaction-treated product of yeast cell wall to the substance having reducing ability is preferably 1 part by weight or more and 900 parts by weight or less to 100 parts by weight.

In addition, the composition of the embodiment may also contain other components in addition to a substance having reducing ability, a wet ball mill-treated product of yeast cell wall and/or a hydrothermal reaction-treated product of yeast cell wall to the extent that the object of the present invention can be achieved, and such components are not particularly limited. For example, the composition may also contain polyphenols, coffee cake, used tea leaves, vitamin C, minerals such as silicon, manganese, iron, and copper, and glutathione, etc., as other components.

As described above, according to the embodiment, a new technique capable of maintaining the reducing ability for a longer period of time can be provided.

Specifically, a wet ball mill-treated product of yeast cell wall and/or a hydrothermal reaction-treated product of yeast cell wall is contained together with a substance having reducing ability such as a gelled quartz porphyry, so that a composition capable of maintaining reducing ability for a longer period of time as compared with a substance having reducing ability such as a gelled quartz porphyry can be provided.

The composition of the embodiment has various uses.

First, the composition of the embodiment has its own reducing ability and can remove active oxygen. Moreover, the composition of the embodiment can donate electrons to a target even if the composition of the embodiment is not in contact with the target such as a beverage or a food through a container material other than aluminum. Therefore, the composition can impart antioxidant activity to a target such as a beverage or a food or enhance the antioxidant activity. As a result, for example, a beverage/food having antioxidant activity imparted thereto or having enhanced antioxidant activity is formed and then ingested by a person or the like, so that an effect of improving lifestyle-related diseases, aging, fatigue, and other physical deconditioning etc., caused by oxidative stress can be expected.

The composition of the embodiment having antioxidant activity can also be used as a material for cosmetics, pharmaceuticals, building materials (inclusion thereof can be expected to make, for example, an effect of improving the oxidative durability of a building).

As one aspect of the present invention, a method for sustaining reducing ability can be provided, which comprises mixing a gelled material obtained by treating a quartz porphyry by wet ball milling, the quartz porphyry containing silicic acid (SiO₂), zinc (Zn), copper (Cu), aluminum (Al), iron (Fe), calcium (Ca), magnesium (Mg), potassium (K), strontium (Sr) and phosphorus (P), and a wet ball mill-treated product of yeast cell wall and/or a hydrothermal reaction-treated product of yeast cell wall.

As one aspect of the present invention, a reducing ability sustaining agent containing a wet ball mill-treated product of yeast cell wall and/or a hydrothermal reaction-treated product of yeast cell wall can be provided.

### Examples

Hereinafter, the present invention will be described in more detail with reference to Examples, but the present invention is not limited thereto.

### [Hydrothermal reaction-treated product of yeast cell wall]

After adding 143.6 g of distilled water to a magnetic stirring hydrothermal reactor, 25.4 g of yeast cell wall (Asahi Food & Healthcare Ltd.) was added. After closing the lid and stirring and mixing, the temperature rise was started. The yeast cell wall was treated for 10 minutes under the conditions of a pressure of 1.6 MPa or more and a temperature of 180°C to obtain the hydrothermal reaction-treated product of yeast cell wall.

### [Composition analysis of quartz porphyry]

Quartz porphyries used were confirmed by fluorescent X-ray analysis to contain silicic acid (SiO₂), zinc (Zn), copper (Cu), aluminum (Al), iron (Fe), calcium (Ca), magnesium (Mg), potassium (K), strontium (Sr), and phosphorus (P). The conditions for fluorescent X-ray analysis are shown below.
Fluorescent X-ray analyzer: ZSX PrimusII manufactured by Rigaku Corporation
X-ray vessel: Rh, measurement spectrum: RhLα, tube voltage: 50 kV, tube current: 60 mA, slit: S2, spectroscopic crystal: Ge, detector: PC, analysis area: 30 mmφ, peak position (20): 89.510 deg., background (20): 87.000 deg. and 92.000 deg., and accumulated time: 60 seconds/sample

### [Preparation of compositions of Examples and Comparative Examples]

Each composition of Examples and Comparative Examples was prepared as follows.
Comparative Example 1: 50 g of distilled water was added to 50 g of quartz porphyry powder, followed by gelling with a wet ball mill (manufactured by KEIKOUSYA).
Comparative Example 2: 49.9 g of distilled water was added to 50 g of quartz porphyry powder, and then 0.1 g of L-ascorbic acid, which is a reducing agent (antioxidant), was added to the product gelled with a wet ball mill.
Example 1: After mixing 50 g of quartz porphyry powder and 5 g of yeast cell wall, 45 g of distilled water was added, and then the mixture was reacted with the use of a wet ball mill for gelling.
Example 2: 45 g of distilled water was added to 50 g of quartz porphyry powder, and then 5 g of a hydrothermal reaction-treated product of yeast cell wall was added to the product gelled with a wet ball mill.
Example 3: 40 g of distilled water was added to 50 g of quartz porphyry powder, and then 10 g of a hydrothermal reaction-treated product of yeast cell wall was added to the product gelled with a wet ball mill.

Each composition of Examples and Comparative Examples was stored in an open state (the upper liquid surface is in contact with oxygen) at room temperature, and changes over time of the redox potential were measured using a redox potential meter (HORIBA). The results are shown in Table 1.

**[Table 1]**

| | Immediately after production | 7 days after production | 15 days after production | 30 days after production |
|---|---|---|---|---|
| Comparative Example 1 | -715mV | +52mV | +76mV | +90mV |
| Comparative Example 2 | -720mV | -87mV | +60mV | +95mV |
| Example 1 | -820mV | -650mV | -765mV | -792mV |
| Example 2 | -805mV | -710mV | -730mV | -733mV |
| Example 3 | -817mV | -755mV | -832mV | -830mV |

As in Table 1, any composition of Examples has a negative redox potential during the test period, and it can thus be understood that the reducing ability is maintained for a very long time as compared with those of Comparative Examples.

## Claims

1. A composition having reducing ability, comprising a gelled material obtained by treating a quartz porphyry by wet ball milling, the quartz porphyry containing silicic acid (SiO₂), zinc (Zn), copper (Cu), aluminum (Al), iron (Fe), calcium (Ca), magnesium (Mg), potassium (K), strontium (Sr), and phosphorus (P), and
a wet ball mill-treated product of yeast cell wall and/or
a hydrothermal reaction-treated product of yeast cell wall.

2. The composition according to claim 1, wherein the composition comprises the hydrothermal reaction-treated product.

3. A method for sustaining reducing ability, comprising forming a composition containing a wet ball mill-treated product of yeast cell wall and/or a hydrothermal reaction-treated product of yeast cell wall together with a gelled material obtained by treating a quartz porphyry by wet ball milling, the quartz porphyry containing silicic acid (SiO₂), zinc (Zn), copper (Cu), aluminum (Al), iron (Fe), calcium (Ca), magnesium (Mg), potassium (K), strontium (Sr), and phosphorus (P).

4. The method according to claim 3, wherein the hydrothermal reaction-treated product is contained together with the gelled material.
